# EUROPÄISCHE PATENTSCHRIFT

(11) **EP 3 624 689 B1**
(45) Veröffentlichungstag und Bekanntmachung des Hinweises auf die Patenterteilung: **16.04.2025**
(21) Anmeldenummer: 18719824.7
(22) Anmeldetag: 19.04.2018
(51) Int. Cl.: A61B 5/11, A61B 5/00, A61F 2/76, A61B 5/107, A61F 2/70

(54) **VERFAHREN ZUM BESTIMMEN VON FEHLSTELLUNGEN IM AUFBAU EINER PROTHESE**
METHOD FOR DETERMINING MALPOSITION IN THE FITTING OF A PROSTHESIS
PROCÉDÉ POUR DÉTERMINER DES MAUVAIS POSITIONNEMENTS DANS L'ASSEMBLAGE D'UNE PROTHÈSE

(30) Priorität: 17.05.2017 DE 102017110762
(43) Veröffentlichungstag der Anmeldung: 25.03.2020
(73) Patentinhaber: Ottobock SE & Co. KGaA, 37115 Duderstadt (DE)
(72) Erfinder: PUSCH, Martin, 37115 Duderstadt (DE); WILL, Christian, 37077 Göttingen (DE); BELLMANN, Malte, 34346 Hann. Münden (DE); ALBRECHT-LAATSCH, Erik, 37124 Rosdorf (DE); SCHMIDT, Martin, 37115 Duderstadt (DE); SZUFNAROWSKI, Filip, 81-856 Gdynia (PL)
(74) Vertreter: Gramm, Lins & Partner Patent- und Rechtsanwälte PartGmbB
(86) Internationale Anmeldenummer: PCT/EP2018/060066
(87) Internationale Veröffentlichungsnummer: WO 2018/210520

(56) Entgegenhaltungen:
- WO-A2-2015/088863
- DE-A1- 102008 024 746
- US-A1- 2006 173 552
- US-A1- 2013 110 011
- US-A1- 2015 182 148
- US-A1- 2015 359 644

## Beschreibung

Die Erfindung betrifft ein Verfahren Einrichten einer Prothese und/oder Orthese und/oder zum Bestimmen von Fehlstellungen im Aufbau einer Prothese und/oder Orthese der unteren Extremität, wobei das Verfahren vollständig von einem Computer oder einer elektrischen Datenverarbeitungseinrichtung durchgeführt wird, mit den Schritten:
a. Aufnehmen von ersten Messdaten wenigstens eines Sensors, der an einem Körpersegment eines Menschen befestigt ist, wobei die ersten Messdaten einem ersten Bewegungszustand des Menschen zugeordnet werden,
b. Aufnehmen von zweiten Messdaten wenigstens eines Sensors, der an einem Körpersegment des Menschen befestigt ist, wobei die zweiten Messdaten einem zweiten Bewegungszustand des Menschen zugeordnet werden und wobei die beiden Bewegungszustände voneinander verschieden sind,
c. Auswerten der ersten und zweiten Messdaten
d. Ermitteln einer Korrekturmaßnahme nach dem Auswerten der ersten und zweiten Messdaten.

Die Erfindung betrifft zudem ein System mit mindestens einem Sensor und einer elektronischen Datenverarbeitungseinrichtung, die eingerichtet ist zum Durchführen eines derartigen Verfahrens.

Der Aufbau einer Prothese, insbesondere einer Beinprothese, muss sorgfältig ausgeführt und insbesondere individuell an den jeweiligen Patienten angepasst werden. Dies gilt sowohl für Oberschenkelamputierte (transfemoral), aber auch für Unterschenkelamputierte (transtibial), die noch über ein natürliches Kniegelenk an dem zu versorgenden Bein verfügen. Selbstverständlich gilt dies auch für andere Prothesen.

Der Prothesenaufbau muss dabei so erfolgen, dass beispielsweise ein möglichst natürliches Gangbild erzeugt wird, sodass der Träger der Prothese keine unbequemen, ungewohnten oder unnatürlichen Bewegungen machen muss, um die Prothese zu steuern. Gleichzeitig muss gewährleistet werden, dass in möglichst vielen Situationen eine möglichst hohe Sicherheit durch die Prothese an ihren Träger vermittelt wird.

Herkömmlicherweise wird ein statischer Aufbau, der an einem stehenden Patienten durchgeführt wird, und ein dynamischer Prothesenaufbau während des Gehens des Patienten durchgeführt. Während für den statischen Aufbau Systeme und Plattformen vorhanden sind, die dem Orthopädietechniker, der den Aufbau vornimmt, objektive Messdaten zur Verfügung stellen, wird der dynamische Aufbau oftmals noch auf der Basis von sehr subjektiven Daten, die der zuständige Orthopädietechniker beispielsweise beim Anschauen des Gangbildes für sich ermittelt, vorgenommen. Dies ist insbesondere dann von Nachteil, wenn beispielsweise aufgrund räumlicher Enge der Orthopädietechniker die nötigen Perspektiven nicht einnehmen kann und beispielsweise den Patienten nicht während des Gehens seitlich betrachten kann.

Um die Qualität des dynamischen Prothesenaufbaus zu verbessern, wäre es von Vorteil, ein System bereitstellen zu können, bei dem unterschiedliche Sensoren, die die benötigten Messwerte aufnehmen können, an unterschiedlichen Stellen des Körpers des Patienten und/oder an unterschiedlichen Positionen der Prothese befestigt werden könnten. Die entsprechenden Sensoren könnten beispielsweise inertiale Daten, wie beispielsweise Absolutwinkel oder Relativwinkel, beispielsweise einen Kniewinkel, Beschleunigungen oder Geschwindigkeiten messen, die objektiv Auskunft über das Gangbild und den vorhandenen Prothesenaufbau liefern. Um verlässliche Daten zu generieren ist es notwendig, die Orientierung des Sensors am Körper oder der Prothese möglichst exakt zu kennen und dafür zu sorgen, dass diese Orientierung und/oder Ausrichtung während des Gehens und er dynamischen Analyse möglichst wenig, vorzugsweise gar nicht, ändert.

Aus der DE 10 2012 009 507 A1 ist beispielsweise ein System und ein Verfahren zur Bestimmung von Fehlstellungen im Aufbau von Prothesen der unteren Extremität bekannt, bei dem inertiale Messdaten und daraus abgeleitete Größen der versorgten Extremität mit entsprechenden Größen der unversorgten Extremität verglichen und auf diese Weise insbesondere Symmetrien erkannt und gegebenenfalls verbessert werden können. Aus der US 2010/0131113 A1 ist ein Verfahren bekannt, bei dem Messdaten aufgenommen werden, indem der Patient gefilmt werden kann. Diese Daten werden anschließend vorzugsweise in Echtzeit mit in einer Datenbank hinterlegten Daten verglichen, in der beispielsweise mögliche Fehlstellungen und deren Effekte auf die Bewegung hinterlegt sind. Die aufgenommenen Messwerte werden folglich mit Sollwerten verglichen um Fehlstellungen aufzuspüren.

Die WO 2015/088863 A2 beschreibt ein Exoskelett, das in Form eines Kleidungsstückes ausgebildet ist und über Aktuatoren und Stützelemente verfügt. Sensoren werden verwendet um die Vorrichtung dem jeweiligen Bewegungszustand und Position und Haltung des Trägers der Vorrichtung entsprechend zu steuern. Da das Kleidungsstück am Körper ausgerichtet wird und Ankerpunkte aufweist, treten Fehlstellungen und Fehlausrichtungen bei dieser Art Unterstützungsvorrichtung nicht auf. Beim Einrichten der Prothese werden Messdaten eines Bewegungszustandes aufgenommen und anhand dieser eine notwendige Anpassung ermittelt und dann vorgenommen. Danach können weitere Messdaten eines anderen Bewegungszustandes aufgenommen und eine weitere Anpassung ermittelt werden. Dabei werden die Messdaten jeweils einzeln gewertet. Sie werden nicht gleichzeitig gewertet, es wird also keine Anpassung ermittelt, die auf der Auswertung beider Sätze von Messdaten beruht, indem beispielsweise die bereits ausgewerteten Messdaten auch bei der Ermittlung der zweiten Anpassung herangezogen werden.

Aus der US 2006/0173552 A1 ist ein aktives Prothesen-Kniegelenk bekannt, das mit Sensoren ausgerüstet ist. Die von diesen Sensoren ermittelten Daten werden verwendet, um das Knie zu steuern.

Die US 2013/0110011 A1 befasst sich mit der Verbesserung des Aufbaus oder der Einrichtung von Prothesen und Orthesen. Dabei werden verschiedene Algorithmen verwendet, um aus Messdaten, die von Sensoren generiert werden, zu ermitteln, ob Fehlstellungen vorliegen und welcher Art sie sind. Dazu werden Messdaten bei einzelnen Bewegungsmustern aufgenommen und ausgewertet, beispielsweise mit Solldaten für diesen speziellen Bewegungsablauf verglichen.

Die Erfahrung zeigt jedoch, dass trotz dieser Verfahren und Methoden ein optimaler Prothesenaufbau nicht oder nur schwer zu erreichen ist. Zudem wird insbesondere bei dem Vergleich aufgenommener Messwerte mit in einer Datenbank hinterlegten Daten eine individuelle Anpassung an die körperlichen Gegebenheiten des Patienten erschwert oder unmöglich gemacht.

Der Erfindung liegt daher die Aufgabe zugrunde, ein Verfahren und ein System anzugeben, mit dem Fehlstellungen im Aufbau einer Prothese besser, objektiver und umfassender aufgedeckt und so entsprechend kompensiert werden können. Unter einer Fehlstellung werden dabei insbesondere alle nicht optimal eingestellten Parameter einer Orthese und/oder Prothese verstanden. Dies kann Positionen einzelner Bauteile zueinander oder zum Körper des Menschen, Bewegungsbereiche, beispielsweise Schwenkbereiche von Gelenken, Dämpfungen, Bewegungswiderstände, aber auch die gegebenenfalls für den jeweils betrachteten Menschen nicht optimale Wahl einzelner Bauteile, beispielsweise eines künstlichen Fußes oder Knies sein.

Die Erfindung löst die gestellte Aufgabe durch ein Verfahren zum Einrichten einer Prothese und/oder Orthese gemäß dem Oberbegriff des Anspruchs 1, das sich dadurch auszeichnet, dass die ersten und zweiten Messdaten gleichzeitig gewertet werden.

Der Erfindung liegt die Erkenntnis zugrunde, dass es zum optimalen Prothesen-aufbau oftmals notwendig ist, Messdaten von Sensoren in unterschiedlichen Bewegungszuständen aufzunehmen und gleichzeitig zu werten. Daher sind der erste Bewegungszustand und der zweite Bewegungszustand voneinander verschieden. Auf diese Weise werden Informationen erhalten, die durch das einfache Auswerten der Messdaten nur eines der beiden Bewegungszustände nicht erhalten werden können. Dabei ist selbstverständlich notwendig, dass die beiden Bewegungszustände voneinander verschieden sind.

Das erfindungsgemäße Verfahren wird vollständig von einem Computer oder einer elektronischen Datenverarbeitungseinrichtung durchgeführt, sofern diese Zugriff auf die aufgenommenen ersten und zweiten Messdaten hat. Diese werden von dem jeweiligen wenigstens einem Sensor der elektronischen Datenverarbeitungseinrichtung übermittelt und in einem elektronischen Daten-speicher vorteilhafterweise abgespeichert.

In einer bevorzugten Ausgestaltung wird der zweite Bewegungszustand auf der Grundlage der ersten Messdaten ausgewählt. Vorzugsweise werden zunächst die ersten Messdaten ausgewertet. Dabei können bereits Indizien für vorhandene Fehl-stellungen ermittelt werden, die durch Messungen in einem zweiten Bewegungszustand verifiziert, bestätigt oder wiederlegt werden können. Es ist daher oftmals von Vorteil, zunächst die ersten Messdaten auszuwählen um den optimalen zweiten Bewegungszustand auszuwählen, sodass mit den zweiten Messdaten die aus den ersten Messdaten noch vorhandenen Unklarheiten und/oder Mehrdeutigkeiten auflösen zu können.

Alternativ dazu ist es selbstverständlich auch möglich, dass einem ausgewählten ersten Bewegungszustand ein fester zweiter Bewegungszustand zugeordnet wird. Ist der erste Bewegungszustand beispielsweise das Treppensteigen nach oben, kann es sinnvoll sein, das Treppensteigen nach unten oder das Gehen auf einer schiefen Ebene, also einer Neigung nach oben oder unten, zuzuordnen. Durch eine derartig feste Zuordnung können eine Vielzahl unterschiedlicher Fehlstellungen aus den Messdaten ermittelt werden. Die feste Zuordnung ist dann sinnvoll, wenn einem ersten Bewegungszustand nur ein einziger zweiter Bewegungszustand zugeordnet werden kann. Sobald hier Mehrdeutigen auftreten und für unterschiedliche Auswerteergebnisse aus den Auswertungen der ersten Messdaten unterschiedliche zweite Bewegungszustände folgen können, ist eine derartige feste Zuordnung nicht mehr sinnvoll.

Für bestimmte Prothesen und/oder Patienten ist es zudem sinnvoll, mehr als zwei Bewegungszustände zu verwenden und in jedem dieser mehreren Bewegungs-zustände separate Messdaten aufzunehmen. In diesem Fall ist es von Vorteil, eine feste Reihenfolge der unterschiedlichen Bewegungszustände zu wählen und die jeweils aufgenommenen Messdaten dem jeweils aktuellen Bewegungszustand zuzuordnen.

Vorzugsweise wird der ausgewählte zweite Bewegungszustand über eine Kommunikationseinrichtung, insbesondere durch ein Audiosignal und/oder ein haptisches Signal und/oder ein visuelles Signal, angezeigt. Eine Vorrichtung, mit der das Verfahren durchgeführt werden kann, verfügt folglich über eine Kommunikationseinrichtung, beispielsweise ein Display oder eine Anzeigeeinrichtung, die beispielsweise in Form von unterschiedlich farbigen LEDs, Lampen oder sonstigen Anzeigeelementen vorliegen kann. Selbstverständlich kann auch ein Lautsprecher vorhanden sein, über den Audiosignale, beispielsweise gesprochene Worte, ausgesandt werden können. Vorzugsweise verfügt die Kommunikationseinrichtung zudem über ein Mikrofon, so dass eine Spracheingabe von Befehlen oder Anweisungen möglich ist. Nachdem die ersten Messdaten ausgewertet wurden und der zweite Bewegungszustand auf der Basis dieser Auswertung ermittelt wurde, wird einem Benutzer der Vorrichtung, beispielsweise einem Orthopädietechniker, über die Kommunikationseinrichtung mitgeteilt, welches der zweite Bewegungszustand ist. Der Patient wird dann beispielsweise durch die Kommunikationseinrichtung oder durch einen Orthopädietechniker aufgefordert, den nächsten Bewegungszustand einzunehmen und beispielsweise Treppen zu steigen, schneller oder langsamer zu gehen, sich hinzustellen oder hinzusetzen. Dabei ist jedoch zu beachten, dass das Verfahren unabhängig von der tatsächlichen Ausführung der unterschiedlichen Bewegungszustände durch den Menschen durchführbar ist. Die nach einer Aufforderung durch die Kommunikationseinrichtung ermittelten Messdaten werden unabhängig davon, ob der zweite Bewegungszustand tatsächlich eingenommen und zutreffend ausgeführt wurde oder nicht, dem zweiten Bewegungszustand zugeordnet. Wie bereits dargelegt, kann das Verfahren vollständig von einem Computer durchgeführt werden, der Zugriff auf die von dem wenigstens einen Sensor aufgenommenen Messdaten hat.

Vorteilhafterweise wird der erste Bewegungszustand aus den ersten Messdaten und/oder der zweite Bewegungszustand aus den zweiten Messdaten erkannt. Dies kann beispielsweise geschehen, in dem bestimmte Messdatenverläufe, beispielsweise ein Kniewinkelverlauf beim Gehen, in einem elektronischen Datenspeicher hinterlegt sind. Die aufgenommenen Messdaten werden anschließend mit einer Reihe dieser unterschiedlichen Bewegungsmuster und Datenverläufe verglichen bis eine Übereinstimmung gefunden wird. Der entsprechende Bewegungszustand wird als erster Bewegungszustand oder zweiter Bewegungszustand erkannt und entsprechend den Messdaten zugeordnet.

Vorzugsweise wird die Korrekturmaßnahme mittels der Kommunikationseinrichtung ausgegeben. Diese Korrekturmaßnahme betrifft den Prothesenaufbau und kann beispielsweise in einer Verschiebung des Fußes relativ zum Unterschenkel und/oder zum Knie, oder einer anderen Positionierung der einzelnen Bauteile der Prothese relativ zueinander bestehen. Die Korrekturmaßnahme kann jedoch zumindest auch oder nur darin bestehen, ein verwendetes Bauteil in der Orthese oder der Prothese gegen ein anderes Bauteil auszutauschen und so beispielsweise einen Prothesenfuß, der in einer Beinprothese verwendet wird, gegen einen anderen Prothesenfuß auszutauschen. Damit kann gewährleistet werden, dass der Träger der Prothese oder Orthese nicht nur eine optimal aufgebaute Prothese oder Orthese, sondern auch eine Prothese oder Orthese aus einer möglichst optimalen Kombination einzelner Bauteile bekommt und verwenden kann.

Vorzugsweise wird die Korrekturmaßnahme durch die Kommunikationseinrichtung an ein Bauteil der Prothese oder Orthese übermittelt. Dies kann kabelgebunden oder kabellos, beispielsweise über Funk, WiFi oder Bluetooth erfolgen. Das Bauteil der Orthese oder Prothese ist vorzugsweise eingerichtet, die entsprechenden Signale umzusetzen und die darin enthaltene Korrekturmaßnahme als Reaktion auf die empfangenen Signale umzusetzen. Dies kann beispielsweise durch eine Erhöhung oder Reduzierung eines Beugewiderstands, eine Veränderung einer Position eines Bauteiles oder eine Einstellung einer Dämpfung geschehen. Sicherheitsrelevante Änderungen und/oder Korrekturmaßnahmen sind vorzugsweise durch einen Menschen, beispielsweise einen Orthopädietechniker zu bestätigen.

Die Korrekturmaßnahme ist vorzugsweise möglichst konkret, so dass sie von einem Orthopädietechniker leicht umgesetzt werden kann. Sie kann beispielsweise Anweisungen enthalten welche Schraube oder welcher Einstellmechanismus wie weit in welche Richtung gedreht oder in welcher Weise eingestellt werden soll.

Zusätzlich oder alternativ zu einer Korrekturmaßnahme kann auch eine Trainingsempfehlung ausgegeben werden, um dem Menschen den optimalen Umgang mit der Prothese oder Orthese zu vermitteln.

Vorzugsweise werden bei dem Auswerten der ersten Messdaten und der zweiten Messdaten technische Eigenschaften und/oder Begrenzungen der Orthese oder Prothese und/oder Bewegungsumfänge und/oder Limitierungen des Menschen berücksichtigt. Kann beispielsweise ein verwendetes Prothesenkniegelenk keine Standphasenbeugung ermöglichen, wird dies beim Auswerten der Messdaten berücksichtigt. Es ist in diesem Fall natürlich nicht sinnvoll, durch eine Korrekturmaßnahme zu versuchen, eine Standphasenbeugung zu erreichen. Wird sie dennoch als vorteilhaft und/oder notwendig angesehen, beinhaltet die Korrekturmaßnahme stattdessen den Austausch des Kniegelenks. Auch Bewegungseinschränkungen, beispielsweise eingeschränkte Bewegungsumfänge, des Menschen werden bevorzugt berücksichtigt um durch das Verfahren einen möglichst optimalen und individuell bestimmten Aufbau der Prothese oder Orthese zu erreichen. Diese Daten werden vorzugsweise in einer Datenbank hinterlegt und können beispielsweise manuell eingegeben werden. Es versteht sich, dass die elektronische Datenverarbeitungseinrichtung auf diese Datenbank Zugriff hat.

Derartige Begrenzungen der verwendeten Bauteile oder des Patienten werden bevorzug vor dem Durchführen des Verfahrens abgefragt und in einer Datenbank hinterlegt, auf die die elektronische Datenverarbeitungseinrichtung Zugriff hat. Alternativ dazu können sie auch durch Datenübermittlung, beispielsweise von einem Speichermedium oder online eingespielt und gespeichert werden.

Es ist von Vorteil, wenn ein biomechanisches Modell des Menschen mit Orthese oder Prothese hinterlegt und anhand der ersten Messdaten und/oder der zweiten Messdaten parametriert wird, das dann verwendet wird, um aus kinematischen Daten kinetische Daten zu berechnen.

Vorteilhafterweise stammen die ersten Messdaten und/oder die zweiten Messdaten zumindest auch von Sensoren, die an einer unversorgten Extremität des Menschen befestigt sind. Vorzugsweise wird ein Teil der gesunden, also unversorgten, Körpersegmenten des Menschen befestigt, während ein anderer Teil an versorgten Körpersegmenten, also an der Prothese selbst befestigt wird. Vorzugsweise wird aus den ersten Messdaten und den zweiten Messdaten eine Symmetrie des Gangbildes ermittelt. Ein Sensor, der an einer Prothese angeordnet wird gilt im Rahmen der vorliegenden Erfindung auch als an einem Körpersegment des Menschen befestigt. Unter einem Körpersegment wird vorliegend sowohl ein natürliches Körperteil eines Menschen als auch ein Bauteil einer Orthese oder Prothese verstanden, die der Mensch trägt.

Geeignete Sensoren sind beispielsweise ein Winkelsensor, Inertialsensor, Drucksensor, Kraft- und/oder Momentensensor, Video- oder Bildsensor. Durch die Kombination von Daten unterschiedlicher Sensoren und/oder unterschiedlicher Bewegungszustände werden neue Erkenntnisse gewonnen, die ohne diese Kombination nicht erhalten werden können. Die Daten unterschiedlicher Quellen werden vorzugsweise fusioniert.

Auf diese Weise können Messdaten sowohl der versorgten als auch der unversorgten Extremität aufgenommen und miteinander verglichen werden. Dies ist insbesondere für Symmetriebetrachtungen und Symmetriemessungen von großem Vorteil. Dies betrifft beispielsweise die Messung der Schrittlänge oder der Doppelschrittlänge sowie der Schrittdauer oder der Doppelschrittdauer. Auch die Ganggeschwindigkeit, der maximale Kniewinkel während der Standphase oder der maximale Kniewinkel während der Schwungphase können auf diese Weise für beide Extremitäten ermittelt und auf Symmetrien miteinander verglichen werden. Sollten hier Asymmetrien erkannt werden, kann dies ein Zeichen für entsprechende Korrekturmaßnahmen sein. Weitere Größen, die durch den wenigstens einen Sensor ermittelt werden können, sind beispielsweise der Zirkumduktionstyp, der vom Patienten ausgeführt wird, die maximale Bodenfreiheit in der Schwungphase eines Schrittes, der minimale Kniewinkel in der Standphase, sowie der prozentuale Anteil der Standphase und/oder der Schwungphase am Gangzyklus, wobei insbesondere die zeitliche Dauer betrachtet wird. Selbstverständlich können auch Kadenz, Streckenlänge und andere Parameter, wie beispielsweise Kniewinkelgeschwindigkeiten, ermittelt werden. Während der Auswertung der Messdaten können aus so ermittelten ursprünglichen Messdaten, die direkt von den Sensoren stammen, weitere abgeleitete Größen, insbesondere zeitliche Ableitungen oder Relativwinkel, ermittelt werden. Alle diese Daten und Messdaten werden vorzugsweise der Auswertung zugrunde gelegt, um einen möglichst optimalen Prothesenaufbau zu gewährleisten.

Vorzugsweise ist der erste Bewegungszustand oder der zweite Bewegungszustand ein Stehen, ein langsames Gehen, ein schnelles Gehen, Treppensteigen aufwärts oder abwärts, das Gehen entlang einer Neigung aufwärts oder abwärts, ein Stehen oder ein Sitzen, ein Aufstehen oder Hinsetzen oder ein Trippeln auf der Stelle (wechselseitiges Heben der Beine). Auch das Beschleunigen ist ein derartiger Bewegungszustand. Vorteilhafterweise unterscheiden sich der erste Bewegungszustand und der zweite Bewegungszustand auch oder nur durch eine Bodenbeschaffenheit. Dabei wird die Bewegung des ersten Bewegungszustandes, beispielsweise Gehen auf der Ebene, auf einem anderen Bodenbelag ausgeführt als die Bewegung des zweiten Bewegungszustandes. Die Bewegungen können beispielsweise auf harten Böden, beispielsweise Stein, Beton oder Holz, oder auf weichen Böden, beispielsweise Sand, Waldboden, Rasen oder Teppichboden durchgeführt werden.

Beim Auswerten der ersten Messdaten und der zweiten Messdaten werden diese vorzugsweise mit hinterlegten Referenzdaten verglichen, die vorzugsweise in einem elektronischen Datenspeicher hinterlegt sind. Dabei kann es sich um Daten anderer Menschen, beispielsweise andere Patienten mit der gleichen Erkrankung oder Behinderung und/oder der gleichen Orthese oder Prothese handeln.

Die Erfindung löst die gestellte Aufgabe zudem mit einem System mit mindestens einem Sensor zum Befestigen an einem Körpersegment eines Menschen und einer elektronischen Datenverarbeitungseinrichtung, die eingerichtet ist zum Durchführen eines hier beschriebenen Verfahrens, wobei das System vorzugsweise eine Kommunikationseinrichtung aufweist, die insbesondere als Display ausgebildet ist. Auch hier gilt, dass ein Sensor, der an einer Prothese oder einem Bauteil der Prothese angeordnet ist, als ein Sensor an einem Körpersegment des Menschen gilt.

Die elektronische Datenverarbeitungseinrichtung ist vorzugsweise eingerichtet, selbstständig einen geeigneten Funktionstest zu ermitteln und durchzuführen.

Bekannte Tests sind beispielsweise ein "Timed up and go test (TUG)", ein "functional reach test (FRT)", eine "2 minutes walk test (2MWT)" oder ein "four square step test (FSST)".

Aus Messdaten, die beim Gehen, das dann den ersten Bewegungszustand bildet, und beim Trippeln, das den zweiten Bewegungszustand bildet aufgenommen wurden lassen sich die Parallelität der Füße (Position der Füße relativ zueinander) sowie die Schrittlängensymmetrie und die Symmetrie der Standphasendauer bestimmen. Sind alle diese Parameter bekannt, die nicht alle aus den Daten nur eines Bewegungszustandes gewonnen werden könne, lässt sich beispielsweise die Flexionskontraktur beurteilen.

Um die Schwungphasenextension und/oder die Schwungphasenflexion beurteil zu können, werden vorzugsweise Messdaten beim langsamen Gehen (erster Bewegungszustand) und beim Beschleunigen (zweiter Bewegungszustand) aufgenommen.

Wird während eine ersten Bewegungszustandes festgestellt, dass der Mensch Fehlstellungen durch andere Bewegungen zu kompensieren versucht, kann der zweite Bewegungszustand auch in einer "entspannten" Durchführung der gleichen Bewegung, beispielsweise Gehen, bestehen.

Vorzugsweise wird das Körpersegment, an dem der wenigstens eine Sensor befestigt ist, durch ein Verfahren erkannt. Dieses Verfahren stellt eine eigenständige Erfindung dar oder kann in Kombination mit anderen hier beschriebenen Merkmalen verwendet werden.

Die Aufgabe wird durch ein Verfahren gelöst, bei dem wenigstens ein Sensor an einem Körpersegment eines Menschen befestigt wird, der wenigstens eine Sensor Signale, die Messdaten und eine individuelle Sensorkennung enthalten, an eine elektronische Datenverarbeitungseinrichtung sendet und die elektronische Datenverarbeitungseinrichtung das Körpersegment bestimmt, an dem der Sensor angeordnet ist oder anzuordnen ist und die Messdaten des wenigstens einen Sensors dem Körpersegment zuordnet. Diese Zuordnung erfolgt vorzugsweise automatisch. Dazu erkennt vorzugsweise die elektronischen Datenverarbeitungseinrichtung die individuelle Sensorkennung.

Unter einem Körpersegment wird vorliegend sowohl ein natürliches Körperteil eines Menschen als auch ein Bauteil einer Orthese oder Prothese verstanden, die der Mensch trägt.

Durch das Verfahren werden die von dem Sensor ausgesandten Signale, die insbesondere die Messdaten enthalten, dem richtigen Körpersegment, an dem sich der Sensor befindet, zugeordnet. Auf diese Weise wird eine objektive Messwerterfassung erreicht, die einen objektiven dynamischen Prothesenaufbau erlaubt und so zu einer besseren Qualität der verschiedenen Prothesen führen wird. Zudem kann der wenigstens eine Sensor unterschiedlichen Körpersegmenten zugeordnet werden, was durch die elektronische Datenverarbeitungseinrichtung erkannt oder ermittelt werden kann, so dass der jeweilige Sensor bei unter-schiedlichen Patienten an unterschiedlichen Körpersegmenten verwendet werden kann. Insbesondere für den Fall, dass mehrere Sensoren an unterschiedlichen Körpersegmenten anzuordnen sind, entfällt dadurch einerseits die Notwendigkeit, eine große Anzahl von Sensoren für die einzelnen Körpersegmente vorzuhalten, und andererseits ein kompliziertes und aufwendiges Verfahren, mit dem sichergestellt werden müsste, dass ein entsprechender Sensor immer an dem dafür vorgesehenen Körpersegment befestigt wird. Dies ist einerseits für den Orthopädietechniker aufwendig und unpraktisch und andererseits fehleranfällig. Diese Nachteile werden durch das erfindungsgemäße Verfahren vermieden.

In einer bevorzugten Ausgestaltung sendet der wenigstens eine Sensor die individuelle Sensorkennung erst nach Aufforderung. Diese Aufforderung erfolgt vor-zugsweise durch ein entsprechenden Steuersignal, dass von der elektronischen Datenverarbeitungseinrichtung ausgesandt und von dem wenigstens einen Sensor detektiert wird. Werden mehrere Sensoren verwendet enthält das Steuersignal vorzugsweise Informationen darüber, welcher Sensor angesprochen wird und seine individuelle Sensorkennung übermitteln soll.

Alternativ kann der wenigstens eine Sensor auch eine Betätigungseinrichtung, beispielsweise einen Druckknopf aufweisen. Wenn die Betätigungseinrichtung betätigt wird, sendet der Sensor seine individuelle Sensorkennung, die von der elektronischen Datenverarbeitungseinrichtung detektiert wird.

In einer bevorzugten Ausgestaltung des Verfahrens ist der elektronischen Datenverarbeitungseinrichtung wenigstens ein Detektor zugeordnet, der zumindest einen Teil der Signale des Sensors detektiert, wobei die elektronische Datenverarbeitungseinrichtung aus den detektierten Signalen, insbesondere aus einer Richtung oder Lage, aus der die individuelle Sondererkennung gesendet wird, oder aus einem zeitlichen Verlauf der Messdaten, das Körpersegment bestimmt.

Die Sensorkennung kann beispielsweise über sichtbares Licht ausgesandt werden. Dies kann vorteilhafterweise über wenigstens eine LED, vorzugsweise mehrere LEDs, die besonders vorzugsweise Licht unterschiedlicher Farben aussenden, gesehen. Die individuelle Sensorkennung kann dabei Kombinationen aus Blinkfrequenz, Farben, Intensität und/oder Muster, eine bestimmte Reihenfolge oder einen Code aus einer oder mehreren dieser Parameter enthalten. Wird die individuelle Sensorkennung von einem Sensor ausgesandt, kann sie von einem entsprechenden Detektor, der der Datenverarbeitungseinrichtung zugeordnet ist und im genannten Ausführungsbeispiel eine Kamera sein kann, erfasst. Selbstverständlich sind auch andere lichtsensitive Sensoren denkbar. Alternativ oder zusätzlich dazu kann die Sensorkennung auch nicht sichtbare elektromagnetische Strahlung, beispielsweise im Infrarotbereich, und/oder hörbare und/oder nicht hörbare akustische Signale, beispielsweise im Ultraschallbereich, enthalten. Entsprechend muss der Detektor in der Lage sein, zumindest einen Teil dieser individuellen Sensorkennung zu detektieren, der es der elektronischen Datenverarbeitungseinrichtung erlaubt, den Sensor anhand der detektierten Teile der individuellen Sensorkennung zu identifizieren. Vorteilhafterweise ist der wenigstens eine Detektor in der Lage, die vollständige Sensorkennung zu detektieren.

Der wenigstens eine Detektor, der der Datenverarbeitungseinrichtung zugeordnet ist, kann jedoch vorteilhafterweise nicht nur die individuelle Sensorkennung, sondern auch weitere Informationen detektieren, aus denen sich die Position des sendenden Sensors ergibt. Dies kann beispielsweise die Richtung sein, aus der die Senderkennung gesendet wird, so dass die elektronische Datenverarbeitungseinrichtung in der Lage ist, das Körpersegment zu bestimmen, an dem der Sensor angeordnet ist. Anschließend weist die elektronische Datenverarbeitungseinrichtung die individuelle Sensorkennung des sendenden Sensors vorzugsweise dem jeweiligen Körpersegment zu, so dass bei der Verwendung des Sensors auch die gesendeten Messdaten als von diesem Sensor kommend identifiziert und dem jeweiligen Körpersegment zugeordnet werden können.

Vorteilhafterweise wird die individuelle Sensorkennung dem jeweiligen Sensor übermittelt, bevor er an dem Körpersegment befestigt wird. Alternativ dazu kann die Kennung auch an den Sender übermittelt werden, wenn dieser bereits an einem Körpersegment des Menschen befestigt wurde. Der wenigstens eine Sensor empfängt seine individuelle Kennung vorteilhafterweise von einer dafür mit der elektronischen Datenverarbeitungseinrichtung verbundenen Sendeeinrichtung und kann vorzugsweise über ein geeignetes Kommunikationsprotokoll der elektronischen Datenverarbeitungseinrichtung eine Bestätigung übermitteln. Aus dieser Bestätigung, die vorteilhafterweise die individuelle Sensorkennung enthält, oder aus den ansonsten übermittelten Signalen kann die elektronische Datenverarbeitungseinrichtung vorteilhafterweise auf die Position und/oder die Richtung, in der sich der Sensor befindet, und damit auf das Körpersegment des Menschen zurückschließen.

Alternativ oder zusätzlich zu diesem Vorgehen kann die elektronische Datenverarbeitungseinrichtung auch aus den detektierten Signalen, beispielswiese dem zeitlichen Verlauf der Messdaten, auf das Körpersegment schließen, an dem sich der Sensor befindet. Dazu ist es von Vorteil, wenn in der elektronischen Datenverarbeitungseinrichtung hinterlegt ist, zu welchem Bewegungsablauf, beispielweise Gehen in der Ebene, Gehen auf schräger Ebene, Treppensteigen, Hinsetzen, Laufen oder Aufstehen, die detektierten zeitlichen Verläufe der Messdaten gehören. Durch Vergleich mit hinterlegten zeitlichen Verläufen von Messdaten kann die elektronische Datenverarbeitungseinrichtung erkennen, an welchem Körpersegment sich der Sensor, zu dem diese detektierten Messdaten gehören, befindet.

Alternativ oder zusätzlich zum Vergleich mit hinterlegten zeitlichen Verläufen von Messdaten, können auch andere Klassifikationsalgorithmen verwendet werden. Dies kann beispielsweise ein simples Einsortieren nach Schwellwerten sein oder ein komplexes selbstständig entscheidendes neurales Netz beinhalten.

Vorzugsweise enthalten die Signale des Sensors zusätzlich Informationen über das Körpersegment, an dem der Sensor befestigt ist. Diese können auf unterschiedlichste Weise eingestellt oder ermittelt werden. In einer besonders bevorzugten Ausgestaltung verfügt der Sensor oder ein Gehäuse, in dem sich der Sensor befindet, über eine Einstelleinrichtung, beispielsweise einen Dreh- oder Schieberegler. Durch Einstellen dieser Einstelleinrichtung werden die in den Signalen des Sensors enthaltenen Informationen über das Körpersegment codiert. Die einzelnen Einstellmöglichkeiten beispielsweise eines Schiebe- oder Drehreglers sind den verschiedenen Körpersegmenten des Menschen zugeordnet, so dass die Person, die den Sensor an dem Körpersegment befestigt hat oder befestigt, die Einstelleinrichtung entsprechend einstellt. Alternativ oder zusätzlich dazu kann der Sensor mittels einer Halterung an dem Körpersegment befestigt sein, die die vom Sensor gesendeten Signale beeinflusst. Die Halterung ist vorzugsweise an das jeweilige Körpersegment des Menschen angepasst. Eine Halterung für einen Sensor, der beispielsweise am Rumpf des Menschen angeordnet werden soll, ist naturgemäß anders ausgestaltet als eine Halterung für einen Sensor, der am Oberarm, am Handgelenk oder am Knie angeordnet werden soll. Da unterschiedliche Halterungen für die unterschiedlichen Körpersegmente verwendet werden müssen, kann in der Halterung beispielweise über einen elektrischen Schaltkreis oder einen RFID-Chip das vom Sensor ausgesandte Signale bezüglich des Körpersegments angepasst werden. Gegebenenfalls ist es von Vorteil, in diesem Fall an der Halterung einen Schalter oder eine Einstelleinrichtung vorzusehen, mit der codiert werden kann, ob die Halterung an einem linken oder einem rechten Körpersegment angeordnet ist.

In einer besonders einfachen Ausgestaltung kann der Sensor die Informationen über das Körpersegment der jeweiligen Halterung entnehmen.

Bevorzugt ermittelt die Halterung das Körpersegment, an dem sie befestigt ist. Dies kann beispielsweise über eine Umfangmessung des Körpersegments geschehen.

Verfügt die Halterung beispielsweise über einen ausziehbaren oder in der Länge verstellbaren Gurt, kann aus der Länge des Gurtes, die benötigt wird, um das Körpersegment zu umgreifen und/oder zu umschließen, ermittelt werden, um welche Art Körpersegment es sich handelt. In diesem Fall müsste beispielsweise lediglich zusätzlich festgelegt werden, ob es sich, sofern das Körpersegment mehrfach vorhanden ist, um eine rechte oder eine linke Ausführung des Körpersegments, beispielsweise eines Beines oder eines Armes, handelt. Alternativ dazu können aufgedruckte Barcodes oder sonstige Codes, Orientierungsmarker, Glyphen oder sonstige Elemente verwendet werden.

Bevorzugt ist die Halterung eingerichtet, die Art des Sensors zu erkennen, der an ihr befestigt ist. So können Informationen über Körpersegmente und/oder Sensoren von der Halterung ermittelt werden und entweder von der Halterung selbst an die elektronische Datenverarbeitungseinrichtung gesandt werden oder über den Sensor versendet werden.

Vorzugsweise verfügt der Sensor über eine Einstelleinrichtung, durch die die Informationen über das Körpersegment eingestellt werden. Damit kann es sich beispielweise um ein manuell betätigbares Element, beispielsweise einen Dreh- oder Schieberegler handeln. Je nach Körpersegment, an dem der Sensor angeordnet wird, werden diese Einstellelemente in eine bestimmte Position und/oder Stellung gebracht, wodurch die Informationen über das Körpersegment, die diese Lage des Sensors enthalten, eingestellt werden.

Vorzugsweise sendet der wenigstens eine Sensor die Signale nur an die elektronische Datenverarbeitungseinrichtung, wenn er ein entsprechendes Abfragesignal erhalten hat. Auf diese Weise kann sichergestellt werden, dass die elektronische Datenverarbeitungseinrichtung nur Signale eines einzelnen Sensors empfängt und nicht mehrere Signale unterschiedlicher Sensoren, die an unterschiedlichen Körpersegmenten des Menschen befestigt sind, einander überlagern und gegebenenfalls nicht ausgewertet werden können.

Vorzugsweise werden mindestens zwei Sensoren an unterschiedlichen Körpersegmenten befestigt.

In einer bevorzugten Ausgestaltung sind diese wenigstens zwei Sensoren eingerichtet, Informationen über Abstände zwischen jeweils zwei dieser Sensoren zu ermitteln und diese in den gesendeten Signalen an die elektronische Datenverarbeitungseinrichtung zu senden. In diesem Fall kann die elektronische Datenverarbeitungseinrichtung aus hinterlegten Mustern von Abständen über die einzelnen Positionen und Körpersegment, an denen die Sensoren befestigt sind, schließen.

Um die Informationen über Abstände zwischen zwei Sensoren zu ermitteln ist es von Vorteil, wenn zumindest einer, vorzugsweise alle der Sensoren in der Lage sind, entsprechende Testsignale, beispielswiese in Form von Vibrationen, elektromagnetische Strahlung oder Schall, auszusenden, die von den jeweils anderen Sensoren detektiert werden können. Es hat sich als vorteilhaft herausgestellt, wenn die Sensoren, die entsprechende Testsignale empfangen, ein entsprechendes Antwortsignal aussenden. Über Laufzeiten des Testsignals und des entsprechenden Antwortsignals kann auf Abstände der verschiedenen Sensoren untereinander und damit auf eine Anordnung der Sensoren relativ zueinander geschlossen werden. Da die möglichen Körpersegmente, an denen Sensoren befestigt werden können, bekannt sind, kann aus diesen Informationen ermittelt werden, welcher Sensor an welchem Körpersegment angeordnet ist. Wird zudem das Testsignal vom ursprünglich sendenden Sensor in eine bestimmte Richtung oder in einen bestimmten Raumbereich gerichtet ausgesandt, kann auch zwischen rechten und linken Körpersegmenten, beispielsweise Beinen oder Armen, unterschieden werden.

Alternativ oder zusätzlich dazu können die Sensoren Testsignale aussenden, die von anderen Sensoren detektiert werden. Informationen über die so detektierten Signale, insbesondere die Detektionszeitpunkte, können dann entweder von den detektierenden Sensoren an die elektronische Datenverarbeitungseinrichtung übermittelt werden oder in Form von Antwortsignalen an den aussendenden Sensor übermittelt werden. Bevorzugt werden sie alternativ oder zusätzlich dazu von der elektronischen Datenverarbeitungseinrichtung empfangen.

Vorteilhafterweise können die Sensoren einen Abstand von einem anderen Gegenstand, beispielsweise dem Boden, bestimmen. Dies kann beispielsweise über Ultraschallsignale erfolgen, die in Richtung des Bodens ausgesandt werden. Die reflektierten Ultraschallsignale können detektiert werden und aus der daraus zu bestimmenden Laufzeit und der bekannten Schallgeschwindigkeit auf den Abstand vom Boden geschlossen werden. Auf diese Weise kann beispielsweise festgestellt werden, ob ein Sensor an einem Knie, einem Oberschenkel oder einer Schulter angeordnet ist. Selbstverständlich können auch andere Körpersegment ermittelt werden, solange diese unterschiedliche Abstände vom Boden aufweisen. Alternativ oder zusätzlich dazu kann auch ein präzise arbeitendes Positionierungssystem, beispielweise ein Global Positioning System (GPS), verwendet werden, um die Position der einzelnen Sensoren möglichst exakt zu bestimmen und so festzustellen, an welchem Körpersegment die Sensoren angeordnet sind.

Zur Bestimmung von Positionen der einzelnen Sensoren relativ zueinander oder relativ zu einem bestimmten Punkt am Körper des Trägers können auch Körpersignale, die vom Menschen ausgehen, verwendet werden. So können beispielsweise der Pulsschlag, die Herzfrequenz, der entsprechende Blutdruck und/oder das EKG verwendet werden, um beispielsweise einen Abstand des Sensors vom Herzen des Menschen zu bestimmen. Selbstverständlich sind alle anderen Biosignale, die vom Körper ausgesandt werden, und eine Orts- und/oder Abstandsbestimmung erlaubt, ebenfalls allein oder in Kombination mit anderen hier aufgeführten Signalen verwendbar.

Eine Alternative bildet ein Verfahren, bei dem wenigstens ein Sensor an einem Körpersegment eines Menschen befestigt wird, der wenigstens eine Sensor Signale, die Messdaten enthalten, an eine elektronische Datenverarbeitungseinrichtung sendet und die elektronische Datenverarbeitungseinrichtung ein Steuersignal an den wenigstens einen Sensor aussendet und der wenigstens eine Sensor als Reaktion auf das Steuersignal ein von einem Menschen detektierbares Antwortsignal aussendet. Dieses Antwortsignal, beispielsweise ein Audiosignal, ein haptisches Signal und/oder ein optisches Signal, wird vom Menschen, beispielsweise einem Orthopädietechniker detektiert, der anschließend vorzugsweise die Position des entsprechenden Sensors der elektronischen Datenverarbeitungseinrichtung mitteilt. Dazu verfügt diese über eine Schnittstelle, durch die ein Mensch entsprechende Daten in die Datenverarbeitungseinrichtung eingeben kann.

Für ein solches Verfahren wird bevorzugt ein System mit wenigstens einem Sensor und einer elektronischen Datenverarbeitungseinrichtung, das eingerichtet ist zum Durchführen eines derartigen Verfahrens, verwendet.

Der wenigstens eine Sensor muss natürlich an einer Prothese oder Orthese befestigt werden. Dann bilden diese Elemente vorzugsweise ein System mit einer Orthese und/oder einer Prothese und einer Halterung zum Befestigen eines Sensors an einem Gegenstand, wobei die Halterung wenigstens zwei Befestigungselemente zum Befestigen der Halterung an dem Gegenstand, die voneinander durch wenigstens ein Abstandselement beabstandet sind, und wenigstens ein Sensorbefestigungselement zum Befestigen des Sensors an der Halterung aufweist, wobei das wenigstens eine Abstandselement in einer ersten Richtung eine kleinere Biegesteifigkeit aufweist als in einer zweiten Richtung, die senkrecht auf der ersten Richtung steht.

Ein solches System stellt eine eigenständige Erfindung dar oder kann in Kombi-nation mit anderen hier beschriebenen Merkmalen verwendet werden. Dies gilt auch für eine Halterung für ein solches System.

Herkömmlicherweise werden Sensoren beispielsweise über einen Gurt, der um ein Körperteil oder ein Prothesenteil herumgelegt wird, an dem jeweiligen Körper-teil oder Prothesenteil befestigt. Dabei sind oftmals Verdrehungen des Sensors relativ zum Körperteil, beispielsweise einem Oberschenkel, möglich, die zur optimalen Auswertung der vom Sensor ermittelten Messdaten erkannt und auch quantifiziert werden müssen. Zudem kann es durch Erschütterungen und Beschleunigungen, die beim Gehen auftreten, zu Verschiebungen oder zum Verrutschen der Sensoren relativ zum Körperteil oder zum Prothesenteil kommen.

Unter einem Körpersegment wird vorliegend sowohl ein natürliches Körperteil eines Menschen als auch ein Bauteil einer Orthese oder Prothese verstanden, die der Mensch trägt.

Durch die erfindungsgemäß vorgesehenen zwei Befestigungselemente, die beispielsweise in Form von zwei Gurten vorliegen können, die um das jeweilige Körperteil oder Prothesenteil herum gelegt werden, wird die Orientierung des zwischen den beiden Befestigungselementen vorhandenen Abstandselementes festgelegt. Eine Rotation oder ein Verschieben dieses Elementes relativ zum Körperteil oder Prothesenteil, beispielsweise relativ zum Oberschenkel, ist nur noch in einem sehr begrenzten Rahmen, vorzugsweise gar nicht mehr möglich. Damit ist die Orientierung zumindest des Abstandselementes relativ zum Gegen-stand, also insbesondere relativ zum Körperteil oder zum Prothesenteil, festgelegt und kann nicht oder nur unwesentlich geändert werden. Der Sensor selbst wird über das Sensorbefestigungselement an der Halterung, besonders bevorzugt am Abstandselement, befestigt. Durch beispielsweise am Sensor oder am Sensorgehäuse, in dem sich der Sensor befindet, vorgesehenen Markierungen lässt sich eine relative Orientierung des Sensorgehäuses oder des Sensors relativ zum Abstandselement leicht ablesen und vorteilhafterweise einstellen. Auf diese Weise kann bereits das Befestigen des Sensors am Gegenstand, also vorzugsweise am Körperteil oder am Prothesenteil, die Ausrichtung und Orientierung des Sensors relativ zu diesem Gegenstand bestimmt und festgelegt werden.

Durch die spezielle Ausgestaltung des Abstandselementes mit der anisotropen Biegesteifigkeit kann gewährleistet werden, dass eine für den Sensor optimale Orientierung beibehalten wird und dennoch eine Bewegung in einer anderen Richtung möglich ist. Ein Sensor kann beispielweise ein Beschleunigungssensor oder ein Winkelsensor sein, der mit einer vorbekannten am Sensor ortsfest vorhandenen Längsachse beispielsweise parallel zum Gegenstand, beispielsweise einem Oberschenkel, ausgerichtet werden sollte, um optimale Messdaten zu erhalten. Dies ist mit einer erfindungsgemäßen Halterung einfach möglich. Die beiden Befestigungselemente werden an zwei voneinander durch das Abstandselement beabstandeten Positionen am Oberschenkel festgelegt. Dabei kann sichergestellt werden, dass sich das Abstandselement parallel zum Oberschenkel, beispielsweise zum Oberschenkelknochen, erstreckt. Der Sensor, der bevorzugt bereits an der Halterung befestigt ist oder nachträglich an ihr befestigt werden kann, hat daher eine vorbekannte vorzugsweise einstellbare Ausrichtung relativ zum Abstandselement und damit relativ zum Oberschenkel. Die gedachte Längsachse des Oberschenkels und die entsprechende festzustellende Richtung des Sensors verlaufen parallel und spannen daher eine Ebene auf. Das Abstandselement verfügt nun in zwei unterschiedlichen Richtungen über unterschiedliche Biegesteifigkeiten, die vorteilhafterweise so gelegt sind, dass die Biegesteifigkeit besonders hoch ist für Biegungen des Abstandselementes, die die vorbestimmte Sensorrichtung aus der gemeinsam mit der Symmetrieachse des Oberschenkels aufgespannten Ebene herausbewegt, so dass eine solche Bewegung, die die Orientierung verändern und damit die Qualität der Messdaten reduzieren würde, nur schwer oder vorzugsweise gar nicht möglich ist. In einer Richtung senkrecht dazu, die vorteilhafterweise die vorbestimmte Richtung des Sensors nicht aus der gemeinsam mit der Symmetrieachse des Oberschenkels aufgespannten Ebene herausführt, ist hingegen deutlich leichter möglich, da hier die Biegesteifigkeit reduziert ist.

Dies ist selbstverständlich nicht auf Ausführungsbeispiele begrenzt, bei denen ein Sensor am Oberschenkel einer Person befestigt wird.

Vorzugsweise ist das wenigstens eine Abstandselement ein Druckkraftübertragungselement. Damit ist sichergestellt, dass die beiden Befestigungselemente immer den gleichen Abstand aufweisen und das Abstandselement drucksteif ist.

In einer bevorzugten Ausgestaltung verfügt das wenigstens eine Abstandselement über wenigstens zwei nebeneinander verlaufende Einzelelemente, insbesondere Stege, Spangen, Stäbe oder Rohre. Auf diese Weise ist es besonders einfach möglich, die unterschiedlichen Biegesteifigkeiten zu erreichen, da die Einzelelemente selbst eine isotrope, also in alle Richtungen gleiche Biegesteifigkeit aufweisen können und allein durch die Positionierung der unterschiedlichen Einzelelemente nebeneinander die gewünschten unterschiedlichen Biegesteifigkeiten des Abstandselementes erreicht werden.

Als besonders vorteilhaft hat sich herausgestellt, wenn die Einzelelemente einstückig miteinander ausgebildet sind. Besonders vorzugsweise ist das gesamte Abstandselement, besonders bevorzugt die gesamte Halterung einstückig ausgebildet. Sie kann beispielsweise ein Kunststoffelement sein, das beispielsweise aus einer Kunststoffplatte herausgeschnitten, herausgesägt oder herausgestanzt wurde. Alternativ können auch 3D-Druckbauteile oder metallische Bauteile verwendet werden. In einer bevorzugten Ausgestaltung sind die Einzelelemente aus einem Kunststoff oder einem Faserverbundwerkstoff, insbesondere einem Kohlefaserverbundwerkstoff oder einem Glasfaserverbundwerkstoff hergestellt. Dies ist insbesondere dann von Vorteil, wenn die Einzelelemente und das Abstandselement nicht einstückig ausgebildet sind. In diesem Fall können Kohlefaserstäbe oder Glasfaserstäbe verwendet werden, die leicht herzustellen sind, eine hohe Biegesteifigkeit aufweisen und dennoch über ein geringes Eigengewicht verfügen.

Vorzugsweise ist das wenigstens eine Sensorbefestigungselement an dem wenigstens einen Abstandselement angeordnet. Als besonders bevorzugt hat sich dabei herausgestellt, wenn das Sensorbefestigungselement in der Mitte zwischen den beiden Befestigungselementen angeordnet ist.

Vorteilhafterweise ist das Sensorbefestigungselement lösbar an dem wenigstens einen Abstandselement angeordnet, bevorzugt angeklemmt oder aufgeklemmt. Auf diese Weise lässt sich beispielsweise besonders leicht der verwendete Sensor austauschen, sofern dies aufgrund der benötigten Messdaten oder zur Wartung und/oder Reparatur des Sensors notwendig ist. Zudem lässt sich erreichen, dass das Sensorbefestigungselement entlang der Längserstreckung des Abstandselementes verschiebbar angeordnet sein kann, so dass auch die Position des Sensors am Gegenstand, also beispielsweise am Körperteil oder am Prothesenteil, nachträglich einstellbar ist und auf die optimale Position gebracht werden kann. Gleichzeitig wird dadurch die Orientierung des wenigstens einen Sensors nicht beeinflusst.

Vorzugsweise verfügt der Gegenstand über eine Längsrichtung und ist insbesondere ein Prothesenbauteil, ein Orthesenbauteil oder ein Segment eines menschlichen Körpers. Vorzugsweise ist die Halterung derart ausgebildet, dass eine Richtung, in der die wenigstens zwei Befestigungselemente voneinander beabstandet sind, der Längsrichtung entspricht oder parallel zu Ihr verläuft, wenn die Halterung an dem Gegenstand angeordnet ist. Diese Richtung entspricht vorteilhafterweise einer Erstreckungsrichtung des Abstandselementes. Wird die Halterung beispielsweise an einem Prothesenbauteil oder einem Segment eines menschlichen Körpers angeordnet, ist auf diese Weise gewährleitet, dass die Orientierung der Halterung und damit vorzugsweise auch die Orientierung des Sensors relativ zu dem Gegenstand festgelegt ist, so dass fehlerhafte Bedienungen und daraus resultierende ungenaue oder falsche Messwerte verhindert oder zumindest verringert werden.

Der wenigstens eine Sensor weist vorzugsweise einen Inertialsensor, einen ins-besondere elektromagnetischen Raumlagesensor, einen Winkelsensor, einen Beschleunigungssensor, einen Sensor zum Detektieren von Biosignalen des menschlichen Körpers, beispielsweise EKG, Blutdruck, Puls oder ähnliches, und/oder einen elektromagnetischen Tracker auf.

Auch ein System aus einer derartigen Halterung sowie einem Sensor, der an dem wenigstens einen Sensorbefestigungselement befestigt ist, bildet eine eigenständige Erfindung, die auch in Kombination mit anderen hier beschriebenen Merkmalen verwendet werden kann. Vorteilhafterweise ist das wenigstens eine Sensorbefestigungselement und der wenigstens eine Sensor derart ausgebildet, dass der Sensor nur in wenigen, vorzugsweise zwei, besonders vorzugsweise nur einer Orientierung an dem Sensorbefestigungselement befestigbar ist. Auf diese Weise ist gewährleistet, dass der Sensor nur in bestimmten Orientierungen befestigbar ist, so dass die Orientierung des Sensors relativ zur Halterung und damit relativ zum Abstandselement oder zum Sensorbefestigungselement nicht detektiert, überprüft und gegebenenfalls eingestellt werden muss und zu-dem keine Quelle von Messfehlern oder sonstigen Ungenauigkeiten ist.

Soll der Prothesenaufbau dynamisch untersucht werden, ist es von Vorteil, Sensoren an unterschiedlichen Stellen und Körpersegmenten eines Menschen anordnen zu können. Eine elektronische Datenverarbeitungseinrichtung ist dann vorzugsweise in der Lage, Messdaten und Signale, die insbesondere auch eine individuelle Sensorkennung beinhalten, dem Körpersegment zuzuordnen, an dem der jeweilige Sensor befestigt ist.

Mit Hilfe der beigefügten Zeichnungen werden nachfolgend Ausführungsbeispiele der vorliegenden Erfindung näher erläutert. Es zeigt:
- Figur 1 -: ein System mit mehreren Sensoren, die Informationen über Bewegungen, Positionen und/oder Orientierungen erfassen können, zum Durchführen eines Verfahrens nach einem ersten Ausführungsbeispiel der vor-liegenden Erfindung,
- Figur 2 -: die schematische Darstellung eines anderen Systems,
- Figur 3 -: die schematische Darstellung eines Beins mit zwei Halterungen gemäß einem Ausführungsbeispiel der vorliegenden Erfindung,
- Figur 4 -: die Darstellung einer Halterung gemäß einem Ausführungsbeispiel der vorliegenden Erfindung,
- Figur 5 -: die Darstellung einer Halterung,
- Figuren 6 und 7-: schematische Details der Halterung aus Figur 5,
- Figuren 8 und 9-: schematische Darstellungen von Befestigungselementen,
- Figur 10 -: die schematische Darstellung einer weiteren Halterung und
- Figur 11 -: die schematische Darstellung eines Verfahrens.

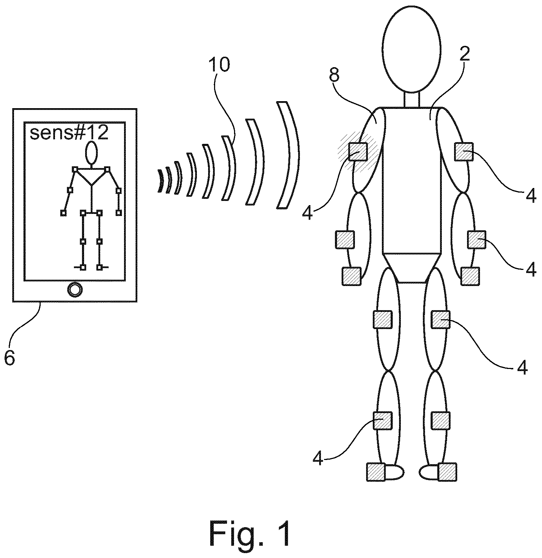

Figur 1 zeigt eine Person 2, an deren Körper eine Mehrzahl von Sensoren 4 befestigt ist. Die Sensoren 4 ermitteln Messwerte beispielsweise Absolutwinkel, Relativwinkel, Geschwindigkeiten oder Beschleunigungen und können diese vorteilhafterweise kabellos an eine elektronische Datenverarbeitungseinrichtung 6 übermitteln. Damit die von den Sensoren 4 ermittelten Messwerte in der elektronischen Datenverarbeitungseinrichtung 6 zutreffend ausgewertet werden können, muss zunächst der Sensor einem Körpersegment 8 zugeordnet werden. Der besseren Übersichtlichkeit wegen ist nur einer Körpersegment 8, nämlich der in Figur 1 links dargestellte Oberarm der Person 2 gekennzeichnet.

Um eine Zuordnung der Sensoren 4 zu den unterschiedlichen Körpersegmenten 8 erreichen zu können, sendet die elektronische Datenverarbeitungseinrichtung 6 im gezeigten Ausführungsbeispiel ein Signal 10, mit dem der an dem Körpersegment 8 angeordnete Sensor 4 zum Aussenden eines Sensorsignals, oder Antwortsignales, stimuliert wird.

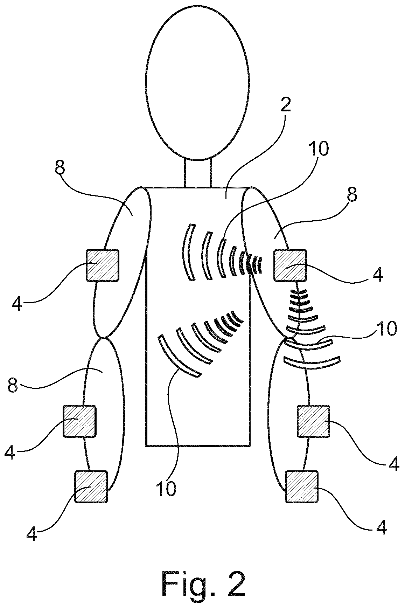

Figur 2 zeigt eine andere Ausgestaltung, bei der die Person 2 nur im Bereich der Arme über Sensoren 4 verfügt. Insbesondere die Sensoren an den Ober- und Unterarmen können durch Halterungen gemäß dem Ausführungsbeispiel der vorliegenden Erfindung, die nachfolgend näher beschrieben dargestellt sind, an den Oberarmen und Unterarmen angeordnet werden. Der in Figur 2 oben rechts am Oberarm dargestellte Sensor 4 sendet Signale 10 aus, die von den anderen Sensoren 4 detektiert werden können. Über die Bestimmung von Laufzeiten lassen sich Abstände zwischen den Sensoren 4 und damit durch Vergleich mit vorbestimmten, berechneten oder gemessenen Mustern die Anordnungen der einzelnen Sensoren 4 an den Körpersegmenten 8 bestimmen.

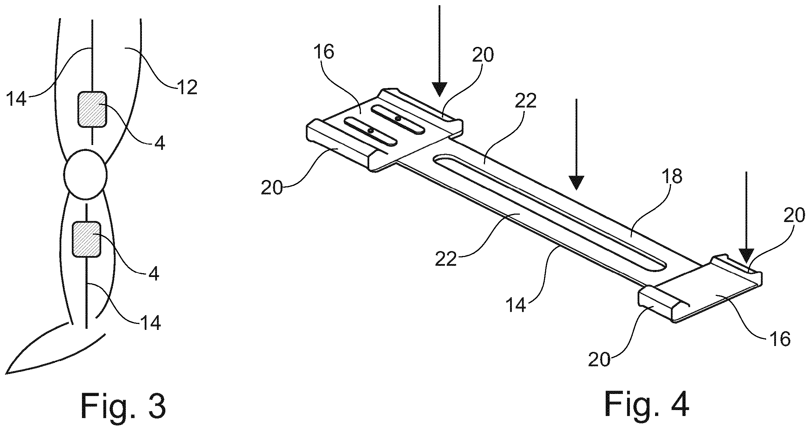

Figur 3 zeigt schematisch ein Bein 12, an dem zwei Sensoren 4 angeordnet sind. Dies geschieht über Halterungen 14, die erfindungsgemäß ausgestaltet sein können und in Figur 3 nur schematisch dargestellt sind.

Figur 4 zeigt eine solche Halterung 14. Sie verfügt über zwei Befestigungselemente 16, die durch ein Abstandselement 18 voneinander getrennt werden. Die beiden Befestigungselemente 16 verfügen über Schlitze 20, durch die beispielsweise ein Befestigungsgurt durchgeführt werden kann, so dass die Halterung 14 am jeweiligen Körpersegment 8 befestigbar ist.

Das Abstandselement 18 verfügt im gezeigten Ausführungsbeispiel über zwei Einzelelemente 22, die nebeneinander angeordnet sind. Dadurch wird erreicht, dass die Biegesteifigkeit des Abstandselementes 18 in einer ersten Richtung deutlich kleiner ist als die Biegesteifigkeit in einer zweiten Richtung.

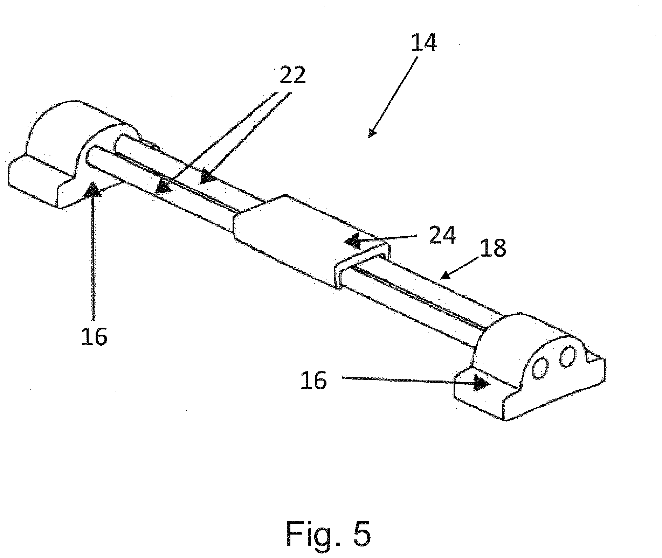

Figur 5 zeigt eine andere Ausgestaltung der Halterung 14. Auch sie verfügt über zwei Befestigungselemente 16, zwischen denen sich das Abstandselement 18 befindet, das wieder über zwei Einzelelemente 22 verfügt. An diesen Einzelelementen 22 befindet sich ein Sensorbefestigungselement 24, das im gezeigten Ausführungsbeispiel entlang der Einzelelemente 22 und damit entlang des Abstandselementes 18 verschiebbar ausgebildet ist.

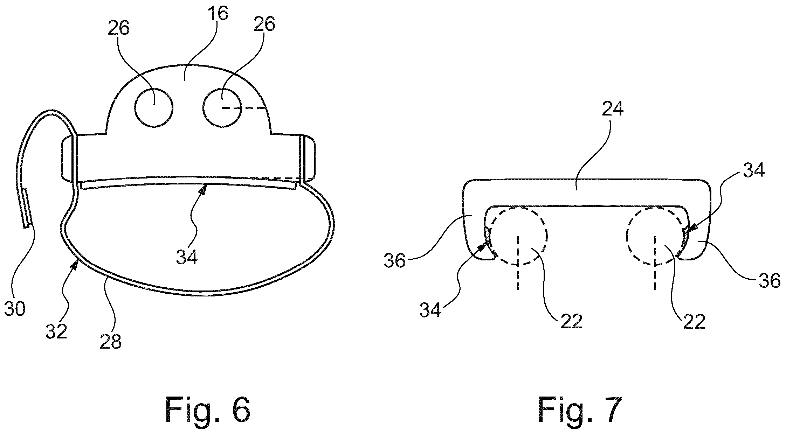

Figur 6 zeigt eine vergrößerte Darstellung aus einer anderen Perspektive eines der Befestigungselemente 16. Man erkennt zwei Öffnungen 26, in die die Einzelelemente 22 eingesteckt sind. Am Befestigungselement 16 befindet sich ein Gurt 28, der um ein Körperteil herumgelegt werden kann. Am freien Ende des Gurtes 28 befindet sich im gezeigten Ausführungsbeispiel ein Klettverschlusselement 30, das an einer Außenseite 32 des Gurtes 28 festgelegt werden kann, so dass der Gurt 28 geschlossen und das Befestigungselement 16 am Körpersegment 8 angeordnet wird. An einer dem Körpersegment 8 zugewandten Seite des Befestigungselementes 16 befindet sich im gezeigten Ausführungsbeispiel eine rutschhemmende Beschichtung 34, durch die ein Verrutschen des Befestigungselementes 16 und damit der Halterung 14 relativ zum Körpersegment 8 verhindert werden soll.

Figur 7 zeigt das Sensorbefestigungselement 24 und die nur gestrichelt dargestellten Einzelelemente 22. Das Sensorbefestigungselement 24 verfügt über zwei Klemmarme 36, die die Einzelelemente 22 wie dargestellt zumindest teilweise umgreifen und so das Sensorbefestigungselement 24 an den Einzelelementen 22 befestigen. Zwischen den Einzelelementen 22 und den Klemmarmen 36 des Sensorbefestigungselementes 24 befindet sich im gezeigten Ausführungsbei-spiel eine rutschhemmende Beschichtung 34, durch die ein versehentliches Verschieben und Verrutschen des Sensorbefestigungselementes 24 relativ zum Abstandselement 18 und dessen Einzelelementen 22 verhindert wird.

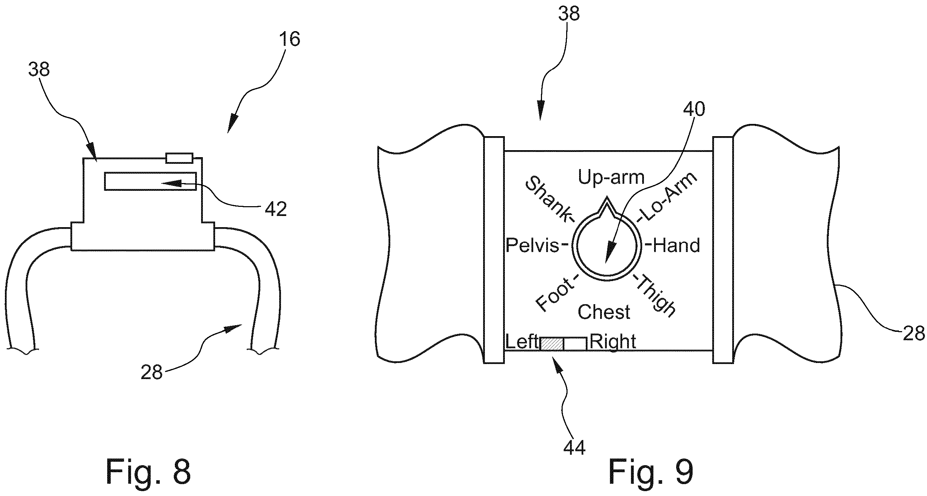

Die Figuren 8 und 9 zeigen ein Befestigungselement 16 in Form eines Gurtes 28. An ihm befindet sich eine Einstelleinrichtung 38, die über einen Drehregler 40 verfügt. Der Drehregler kann je nach Körpersegment 8, an dem ein Sensor mit diesem Befestigungselement 16 angeordnet wird, einstellen. Die entsprechende Kennung wird auf einem kleinen Display 42 dargestellt. über einen Schieberegler 44 lässt sich im gezeigten Ausführungsbeispiel zudem einstellen, ob ein Sensor 4, der an einer Halterung 14 angeordnet wird, die mit einem derartigen Befestigungselement 16 ausgerüstet ist, an einer rechten oder linken Körperseite an-geordnet ist.

Durch die Einstellung des Drehreglers 40 und des Schiebereglers 44 lässt sich beispielsweise ein Signal, das vom jeweiligen Sensor 4 auf ein Aufforderungssignal 10, wie es beispielsweise in Figur 1 schematisch dargestellt ist, aussendet, einstellen. Auf diese Weise können identische Sensoren für beispielsweise unterschiedliche Patienten an unterschiedlichen Orten und Körpersegmenten 8 verwendet werden, ohne dass die Sensoren oder die elektronische Datenverarbeitungseinrichtung eingestellt werden müssen.

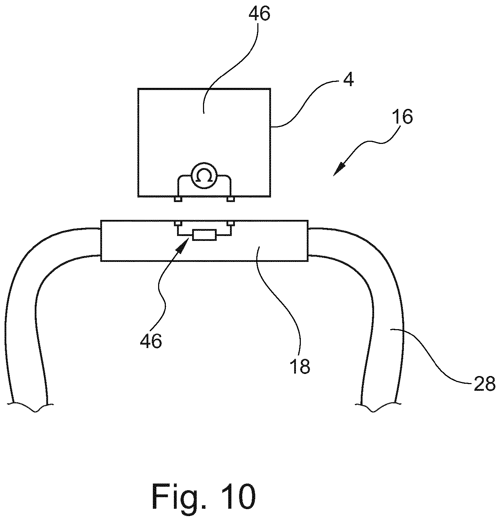

Figur 10 zeigt eine weitere Ausgestaltung eines Befestigungselementes 16, das wieder über einen Gurt 28 verfügt. Zudem ist in einer Seitenansicht das Abstandselement 18 dargestellt, das im gezeigten Ausführungsbeispiel so ausgebildet ist, dass der Sensor 4 direkt am Abstandselement 18 angeordnet werden kann. Dabei verfügt sowohl das Abstandselemente 18 als auch der Sensor 4 über eine elektronische Baugruppe 46, die durch Anordnen des Sensors 4 am Abstandselement 18 in elektrischen Kontakt gebracht werden. Auf diese Weise wird ein Signal, das der Sensor 4 zu einer elektronischen Datenverarbeitungseinrichtung 6 aussendet, modifiziert, so dass die elektronische Datenverarbeitungseinrichtung den Signalinformationen über die Art des Sensors 4 und/oder die Position und Orientierung des Sensors 4, also insbesondere das Körpersegment 8 entnehmen kann.

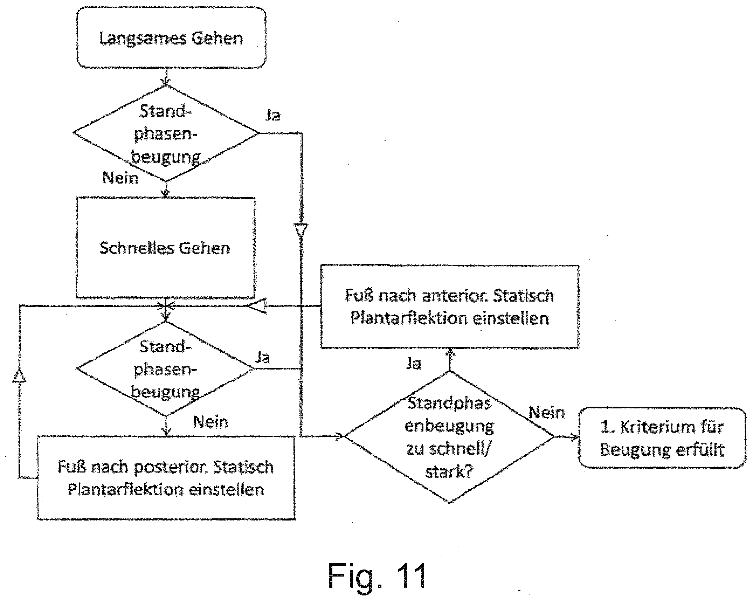

Figur 11 zeigt den schematischen Ablauf eines Verfahrens gemäß einem Ausführungsbeispiel der vorliegenden Erfindung. Im vorliegenden Verfahren soll geprüft werden, ob ein Prothesenfuß in anteriorposteriore Richtung richtig positioniert ist oder ob er in anteriore oder posteriorer Richtung verschoben werden muss. Dazu werden im ersten Verfahrensschritt, der mit "langsames Gehen" gekennzeichnet ist, die ersten Messdaten aufgenommen. Dabei handelt es sich vorzugsweise um den Kniewinkel, also dem Winkel zwischen dem Oberschenkel und dem Unterschenkel. Dies kann beispielsweise über zwei Inertialwinkelsensoren, die den Winkel des Oberschenkels relativ zur vertikalen und den Winkel des Unterschenkels relativ zur vertikalen messen, geschehen. Die Vertikale ist dabei die Richtung entlang der Schwerkraft. Anschließend werden die so aufgenommenen ersten Messdaten dahingehend ausgewertet, ob eine Standphasenbeugung vorliegt. Sofern dies nicht der Fall ist, werden die zweiten Messdaten in einem weiteren Verfahrensschritt, der mit "schnelles Gehen" gekennzeichnet ist, aufgenommen. Das schnelle Gehen bildet dabei den zweiten Bewegungszustand. Langsames Gehen und schnelle Gehen sind dabei zunächst nicht auf bestimmte Geschwindigkeitsbereiche des Gehens eingeschränkt. Durch die Bezeichnungen wird jeweils lediglich dargestellt, dass der erste Bewegungszustand " langsames Gehen" eine kleinere Vorwärtsgeschwindigkeit aufweist als der zweite Bewegungszustand "schnelles Gehen". Das Gehen selbst findet dabei auf einer Ebene statt, die vorzugsweise senkrecht auf der Vertikalen, also entlang der Horizontalen, steht. Eine Neigung weist diese Ebene vorteilhafterweise nicht auf.

Auch die so aufgenommenen zweiten Messdaten werden daraufhin untersucht, ob eine Standphasenbeugung vorliegt. Bei einer Standphasenbeugung handelt es sich um eine Beugung des Knies während der ersten Hälfte der Standphase eines Gangzyklus. Dabei kommt es nach dem Fersenauftritt zu einer kurzfristigen Verringerung des Kniewinkels, also zu einem Einbeugen des Knies. Sofern dies in beiden Bewegungszuständen, also in beiden aufgenommenen Messdaten nicht vorhanden ist, wird vorgeschlagen, den Prothesenfuß in posteriore Richtung zu verschieben. Anschließend sollte in einem statischen Aufbau die Plantarflexion eingestellt werden. Kann jedoch aus den ersten Messdaten und/oder den zweiten Messdaten eine Standphasenbeugung detektiert werden, muss überprüft werden, ob diese in einem angemessenen Geschwindigkeitsbereich stattfindet und eine ausreichende Stärke aufweist. Eine zu schnelle Standphasenbeugung oder eine zu starke Standphasenbeugung sind ebenfalls auf Fehl-stellungen im Prothesenaufbau zurückzuführen, die korrigiert werden müssen. Um dies zu tun wird vorgeschlagen, den Fuß nach anterior zu verschieben und anschließend vorzugsweise die Plantarflexion statisch einzustellen. Dieses Verfahren wird solange durchlaufen, bis das Beugungskriterium erfüllt ist und die anterior-posteriore Ausrichtung des Prothesenfußes ausreichend bestimmt ist. Dieses Verfahren wird vorzugsweise bei transtibial amputierten Patienten, die folglich ein natürliches Knie aufweisen, durchgeführt.

### Bezugszeichenliste

- 2: Person
- 4: Sensor
- 6: elektronische Datenverarbeitungseinrichtung
- 8: Körpersegment
- 10: Signal
- 12: Bein
- 14: Halterung
- 16: Befestigungselement
- 18: Abstandselement
- 20: Schlitz
- 22: Einzelelement
- 24: Sensorbefestigungselement
- 26: Öffnung
- 28: Gurt
- 30: Klettverschlusselement
- 32: Außenseite
- 34: rutschhemmende Beschichtung
- 36: Klemmarm
- 38: Einstelleinrichtung
- 40: Drehregler
- 42: Display
- 44: Schieberegler
- 46: elektronische Baugruppe

## Patentansprüche

1. Verfahren zum Einrichten einer Prothese und/oder Orthese oder zum Bestimmen von Fehlstellungen im Aufbau einer Prothese und/oder Orthese der unteren Extremität, wobei das Verfahren vollständig von einem Computer oder einer elektrischen Datenverarbeitungseinrichtung durchgeführt wird, mit den Schritten:
a. Aufnehmen von ersten Messdaten wenigstens eines Sensors, der an einem Körpersegment eines Menschen befestigt ist, wobei die ersten Messdaten einem ersten Bewegungszustand des Menschen zugeordnet werden,
b. Aufnehmen von zweiten Messdaten wenigstens eines Sensors, der an einem Körpersegment des Menschen befestigt ist, wobei die zweiten Messdaten einem zweiten Bewegungszustand des Menschen zugeordnet werden und wobei die beiden Bewegungszustände voneinander verschieden sind,
c. Auswerten der ersten und zweiten Messdaten
d. Ermitteln einer Korrekturmaßnahme nach dem Auswerten der ersten und zweiten Messdaten,
**dadurch gekennzeichnet, dass** die ersten und zweiten Messdaten gleichzeitig gewertet werden.

2. Verfahren nach Anspruch 1, **dadurch gekennzeichnet, dass** der zweite Bewegungszustand auf der Grundlage der ersten Messdaten ausgewählt wird.

3. Verfahren nach Anspruch 2, **dadurch gekennzeichnet, dass** der ausgewählte zweiten Bewegungszustand über eine Kommunikationseirichtung, insbesondere durch ein Audiosignal und/oder ein haptisches Signal und/oder ein visuelles Signal, angezeigt wird.

4. Verfahren nach einem der vorstehenden Ansprüche, **dadurch gekennzeichnet, dass** der erste Bewegungszustand aus den ersten Messdaten und/oder der zweite Bewegungszustand aus den zweiten Messdaten erkannt wird.

5. Verfahren nach einem der vorstehenden Ansprüche, **dadurch gekennzeichnet, dass** die Korrekturmaßnahme mittels der Kommunikationseinrichtung ausgegeben wird, wobei bevorzugt die Korrekturmaßnahme zumindest auch in einem Austausch wenigstens eines Bauteils der Orthese oder Prothese bestehen kann.

6. Verfahren nach Anspruch 5, **dadurch gekennzeichnet, dass** die Korrekturmaßnahme durch die Kommunikationseinrichtung an ein Bauteil der Orthese und/oder Prothese übermittelt wird, das eingerichtet ist, als Reaktion auf die so übermittelten Signale die Korrekturmaßnahme durchzuführen.

7. Verfahren nach einem der vorstehenden Ansprüche, **dadurch gekennzeichnet, dass** bei dem Auswerten der ersten und zweiten Messdaten technische Eigenschaften und/oder Begrenzungen der Orthese oder Prothese und/oder Bewegungsumfänge und/oder Limitierungen des Menschen berücksichtigt werden.

8. Verfahren nach einem der vorstehenden Ansprüche, **dadurch gekennzeichnet, dass** die ersten Messdaten und/oder die zweiten Messdaten durch mehrere Sensoren aufgenommen werden, wobei vorzugsweise für die ersten Messdaten und die zweiten Messdaten die gleichen Sensoren verwendet werden.

9. Verfahren nah einem der vorstehenden Ansprüche, **dadurch gekennzeichnet, dass** die ersten Messdaten und/oder die zweiten Messdaten auch von Sensoren stammen, die an einer unversorgten Extremität des Menschen befestigt sind, wobei vorzugsweise aus den ersten Messdaten und den zweiten Messdaten eine Symmetrie eines Gangbildes ermittelt wird.

10. Verfahren nach einem der vorstehenden Ansprüche, **dadurch gekennzeichnet, dass** der erste Bewegungszustand oder der zweite Bewegungszustand langsames Gehen, schnelles Gehen, Treppensteigen, Gehen entlang einer Neigung oder Stehen ist.

11. Verfahren nach einem der vorstehenden Ansprüche, **dadurch gekennzeichnet, dass** sich der erste Bewegungszustand von dem zweiten Bewegungszustand zumindest auch durch eine Bodenbeschaffenheit unterscheidet.

12. Verfahren nach einem der vorstehenden Ansprüche, **dadurch gekennzeichnet, dass** die ersten und/oder zweiten Messdaten beim Auswerten der ersten und zweiten Messdaten mit Referenzdaten verglichen werden, die bevorzugt in einem elektronischen Datenspeicher hinterlegt sind.

13. System mit mindestens einem Sensor zum Befestigen an einem Körpersegment eines Menschen und einer elektronischen Datenverarbeitungseinrichtung, die eingerichtet ist zum Durchführen eines Verfahrens nach einem der vorstehenden Ansprüche, wobei das System vorzugsweise wenigstens eine Kommunikationseinrichtung aufweist, die insbesondere als Display ausgebildet ist.

14. System nach Anspruch 13, **dadurch gekennzeichnet, dass** die elektronische Datenverarbeitungseinrichtung eingerichtet ist, das Körpersegment zu bestimmen, an dem der Sensor befestigt ist.

## Claims

1. A method for configuring a prosthesis and/or orthosis or for determining mal-positions in the mounting of a prosthesis and/or orthosis of the lower limb, wherein the method is conducted entirely by a computer or an electric data processing device, featuring the steps:
a. Recording a first set of measurement data of at least one sensor that is fixed to a body part of a person, wherein the first set of measurement data is allocated to a first state of movement of the person,
b. Recording a second set of measurement data of at least one sensor that is fixed to a body part of a person, wherein the second set of measurement data is allocated to a second state of movement of the person, wherein the two states of movement differ from one another
c. Evaluating of the first and second set of measurement data,
d. Detecting a corrective measure after evaluating the first and second set of measurement data,
**characterized in that** the first and second set of measurement data are evaluated simultaneously.

2. The method according to claim 1, **characterized by** the fact that the second state of movement is selected on the basis of the first set of measurement data.

3. The method according to claim 2, **characterized by** the fact that the selected second state of movement is displayed via a communication device, especially by way of an audio signal and/or a haptic signal and/or a visual signal.

4. The method according to one of the above claims, **characterized by** the fact that the first state of movement is recognized from the first set of measurement data and/or the second state of movement from the second set of measurement data.

5. The method according to one of the above claims, **characterized by** the fact that the corrective measure is emitted by means of the communication device, wherein the corrective measure may preferably at least also constitute an exchange of at least one component of the orthosis or prosthesis.

6. The method according to claim 5, **characterized by** the fact that the corrective measure is transmitted by the communication device to a component of the orthosis and/or prosthesis, said component being configured to carry out the corrective measure in response to the thus transmitted signals.

7. The method according to one of the above claims, **characterized by** the fact that technical properties and/or restrictions of the orthosis or prosthesis and/or movement ranges and/or limitations of the person are taken into account during the evaluation of the first and second set of measurement data.

8. The method according to one of the above claims, **characterized by** the fact that the first set of measurement data and/or the second set of measurement data is recorded by several sensors, wherein it is preferable if the same sensors are used for the first and second set of measurement data.

9. The method according to one of the above claims, **characterized by** the fact that the first and/or second set of measurement data also originates from sensors that are fixed to an untreated limb of the person, wherein a symmetry of a gait pattern is preferably determined from the first and second set of measurement data.

10. The method according to one of the above claims, **characterized by** the fact that the first state of movement or the second state of movement is slow walking, fast walking, climbing stairs, walking on an incline or standing.

11. The method according to one of the above claims, **characterized by** the fact that the first state of movement at least also differs from the second state of movement by way of a ground condition.

12. The method according to one of the above claims, **characterized by** the fact that, upon evaluation of the first and second set of measurement data, the first and/or second set of measurement data is compared with reference data that is preferably stored in an electronic memory.

13. A system with at least one sensor to be fixed to a body part of a person and an electronic data processing device, which is configured to conduct a method according to one of the above claims, wherein the system preferably has at least one communication device, particularly one which is designed as a display.

14. The system according to claim 13, **characterized by** the fact that the electronic data processing device is configured to determine the body part to which the sensor is fixed.

## Revendications

1. Procédé d'ajustement d'une prothèse et/ou d'une orthèse ou de détermination de défauts de positionnement dans la structure d'une prothèse et/ou d'une orthèse du membre inférieur, le procédé étant mis en œuvre complètement par un ordinateur ou par un dispositif électrique de traitement de données, comprenant les étapes consistant à :
a. enregistrer des premières données de mesure d'au moins un capteur fixé à un segment du corps d'un être humain, les premières données de mesure étant associées à un premier état de mouvement de l'être humain,
b. enregistrer des deuxièmes données de mesure d'au moins un capteur fixé à un segment du corps de l'être humain, les deuxièmes données de mesure étant associées à un deuxième état de mouvement de l'être humain, et les deux états de mouvement étant différents l'un de l'autre,
c. évaluer les premières et deuxièmes données de mesure,
d. déterminer une action de correction, une fois que les premières et deuxièmes données de mesure sont évaluées,
**caractérisé en ce que** les premières et deuxièmes données de mesure sont évaluées simultanément.

2. Procédé selon la revendication 1,
**caractérisé en ce que** le deuxième état de mouvement est sélectionné sur la base des premières données de mesure.

3. Procédé selon la revendication 2,
**caractérisé en ce que** le deuxième état de mouvement sélectionné est indiqué par l'intermédiaire d'un dispositif de communication, en particulier par un signal audio et/ou un signal haptique et/ou un signal visuel.

4. Procédé selon l'une des revendications précédentes,
**caractérisé en ce que** le premier état de mouvement est reconnu à partir des premières données de mesure et/ou le deuxième état de mouvement est reconnu à partir des deuxièmes données de mesure.

5. Procédé selon l'une des revendications précédentes,
**caractérisé en ce que** l'action de correction est émise au moyen du dispositif de communication, de préférence l'action de correction pouvant également consister au moins en un remplacement d'au moins un composant de l'orthèse ou de la prothèse.

6. Procédé selon la revendication 5,
**caractérisé en ce que** l'action de correction est transmise par le dispositif de communication à un composant de l'orthèse et/ou de la prothèse qui est agencé pour effectuer l'action de correction en réponse aux signaux ainsi transmis.

7. Procédé selon l'une des revendications précédentes,
**caractérisé en ce que** des caractéristiques techniques et/ou des limitations de l'orthèse ou de la prothèse et/ou des amplitudes de mouvement et/ou des restrictions de l'être humain sont prises en compte lors de l'évaluation des premières et deuxièmes données de mesure.

8. Procédé selon l'une des revendications précédentes,
**caractérisé en ce que** les premières données de mesure et/ou les deuxièmes données de mesure sont enregistrées par plusieurs capteurs, de préférence les mêmes capteurs étant utilisés pour les premières données de mesure et les deuxièmes données de mesure.

9. Procédé selon l'une des revendications précédentes,
**caractérisé en ce que** les premières données de mesure et/ou les deuxièmes données de mesure proviennent également de capteurs fixés à un membre, non équipé, de l'être humain, de préférence une symétrie d'un schéma de marche étant déterminée à partir des premières données de mesure et des deuxièmes données de mesure.

10. Procédé selon l'une des revendications précédentes,
**caractérisé en ce que** le premier état de mouvement ou le deuxième état de mouvement est la marche lente, la marche rapide, la montée d'escaliers, la marche le long d'une pente, la position debout.

11. Procédé selon l'une des revendications précédentes,
**caractérisé en ce que** le premier état de mouvement se distingue du deuxième état de mouvement au moins également par la nature du sol.

12. Procédé selon l'une des revendications précédentes,
**caractérisé en ce que** lors de l'évaluation des premières et deuxièmes données de mesure, les premières et/ou deuxièmes données de mesure sont comparées à des données de référence qui sont de préférence stockées dans une mémoire de données électronique.

13. Système comprenant au moins un capteur, destiné à être fixé sur un segment du corps d'un être humain, et un dispositif électronique de traitement de données agencé pour mettre en œuvre un procédé selon l'une des revendications précédentes, le système présentant de préférence au moins un dispositif de communication qui est en particulier conçu comme un écran d'affichage.

14. Système selon la revendication 13,
**caractérisé en ce que** le dispositif électronique de traitement de données est agencé pour déterminer le segment de corps auquel le capteur est fixé.
